(19) Europäisches Patentamt

European Patent Office

Office européen des brevets

(11) Veröffentlichungsnummer: **0 157 371**
A2

(12) # EUROPÄISCHE PATENTANMELDUNG

(21) Anmeldenummer: 85103754.9

(22) Anmeldetag: 28.03.85

(51) Int. Cl.⁴: **A 61 K 35/74,** C 12 P 1/04
// (C12P1/04, C12R1:44)

(30) Priorität: 04.04.84 DE 3412589

(43) Veröffentlichungstag der Anmeldung: 09.10.85
Patentblatt 85/41

(84) Benannte Vertragsstaaten: **AT BE CH DE FR GB IT LI LU NL SE**

(71) Anmelder: **Peters, Georg, Dr. med., Ginsterweg 10, D-5000 Köln 40 (DE)**
Anmelder: **Pulverer, Gerhard, Prof. Dr. med., Mohnweg 25, D-5000 Köln 40 (DE)**

(72) Erfinder: **Peters, Georg, Dr. med., Ginsterweg 10, D-5000 Köln 40 (DE)**
Erfinder: **Pulverer, Gerhard, Prof. Dr. med., Mohnweg 25, D-5000 Köln 40 (DE)**

(74) Vertreter: **Werner, Hans-Karsten, Dr. et al, Deichmannhaus am Hauptbahnhof, D-5000 Köln 1 (DE)**

(54) Arzneimittel mit immunmodulierender und die Proliferation von malignen Zellen hemmender Wirkung und Verfahren zu seiner Herstellung.

(57) Arzneimittel mit immunmodulierender und die Proliferation von malignen Zellen hemmender Wirkung enthaltend eine im Schleim von Staphylokokken vorhandene extrazelluläre Substanz, welche aus lyophilisierter roher Schleimsubstanz gewonnen werden kann.

0157371

Gegenstand der vorliegenden Erfindung sind Arzneimittel mit immunmodulierender und die Proliferation von malignen Zellen hemmender Wirkung und Verfahren zu ihrer Herstellung.

Es ist bekannt, daß an Plastikimplantaten, insbesondere an Unebenheiten der Oberfläche koagulasenegative Staphylokokken angelagert und nur sehr schwer wieder entfernt werden können, da sie sich obendrein mit einer Schleimsubstanz überziehen, die offensichtlich für Antibiotika und natürliche Abwehrstoffe praktisch undurchdringbar ist; vgl. Zbl. Bakt. Hyg., B 173, S. 285-292 und 293-299 sowie S. 300-307 (1981), ferner B 177, S. 527-532 (1983). Weiterhin wurde gefunden, daß diese Schleimsubstanz die menschliche zelluläre Immunreaktion beeinflussen kann, beispielsweise durch die Inhibition der lymphoproliferativen Antwort mononukleärer Zellen auf polyklonale Stimulation; vgl. The Lancet, 1984 S. 365-367.

Es wurde jetzt gefunden, daß tatsächlich im Schleim von Staphylokokken eine extrazelluläre Substanz vorhanden ist, die immunmodulierende und die Proliferation von malignen Zellen hemmender Wirkung aufweist. Diese Substanz moduliert beispielsweise Lymphozyten, insbesondere T-Lymphozyten. Sie vermag weiterhin die zelluläre Immunabwehr zu unterdrücken und damit die Transplantatabstoßung zu verhindern. Sie verhindert oder unterbindet eine allergische Reaktion vom zellvermittelten Typ (Typ IV). Schließlich weist sie eine Wirkung auf maligne proliferierende Lymphozyten (Granulozyten?, Plasmazellen?) oder andere maligne Zellen auf, insbesondere von soliden Tumoren. Die Strukturenzusammensetzung dieser extrazellulären Substanz ist noch nicht geklärt. Die Substanz ist enthalten in einer Fraktion des

0157371

Schleimes, die dadurch erhältlich ist, daß die lyophilisierte rohe Schleimsubstanz

a) in Kochsalzlösung gelöst und mit Phenol-Kochsalz bei 65°C extrahiert wird,

b) die wässrige Phase dialysiert und lyophilisiert in Phosphatpuffer gelöst auf einer DEAE-Sepharose Cl-6B-Säule mit einem Kochsalzgradienten eluiert wird, und

c) die letzte (4.) bei 220 nm absorbierende Fraktion getrennt aufgefangen, dialysiert, ultrafiltriert und lyophilisiert wird. Eine Untersuchung dieser Fraktion hat ergeben, daß sie Galaktose, Ribose, Glukose, Aminozucker und Glukuronsäure enthält, eine positive, spezifische Präzipitationsreaktion mit Poly-L-Lysin, aber keine Präzipitation mit Mannose-spezifischen Lektinen aufweist.

Es wird angenommen, daß die Aktivität dieser Fraktion auf eine oder mehrere extrazelluläre Substanzen im Schleim von Staphylokokken zurückzuführen ist. Dazu ist es erforderlich, diese Fraktion ihrerseits in weitere Unterfraktionen aufzuteilen und diese auf ihre Wirksamkeit hin zu untersuchen. Auch dann wird es erst möglich sein, die Strukturformel der wirksamen extrazellulären Substanz zu bestimmen. Da die immunmodulierende und die Proliferation von malignen Zellen hemmender Wirkung jedoch bereits in der oben beschriebenen Fraktion vorhanden und angereichert ist, kann diese bereits für die Herstellung von Arzneimitteln verwendet werden.

Die erfindungsgemäßen Arzneimittel können in üblicherweise als Injektionslösungen und Infusionslösungen vorliegen. Insbesondere bei magensaftresistenter Dragierung können sie aber auch als orale Mittel vorliegen. Weiterhin ist die Aufnahme durch die Schleimhäute und die Haut möglich, so daß hierfür geeignete Applikationsformen in Frage kommen.

Die Wirksamkeit des erfindungsgemäßen Arzneimittels tritt im Gegensatz zu anderen Wirkstoffen erst nach einigen Tagen in Erscheinung, zeigt danach aber eine langanhaltende Wirkung, so daß diese Wirkung entsprechend therapeutisch zum Tragen kommt.

In den nachfolgenden Beispielen sind die Gewinnung und Anreicherung sowie die Verwendung der extrazellulären Substanz aus dem Schleim von Staphyolokokken näher beschrieben.

## B e i s p i e l 1

Die als Ausgangssubstanz verwendete extrazelluläre Staphylokokken-Schleimsubstanz wird nach einer der drei folgenden Methoden hergestellt:

a) Vorkultur des Staphylokokken-Stammes in Brain Heart Infusion-Bouillon (BHI) über Nacht bei 37°C. Die Staphylokokken-Zellen werden abzentrifugiert, dreimal in physiologischer Kochsalzlösung gewaschen und schließlich darin resuspendiert ($\rightarrow \sim 10^8$ Kolonie-bildende-Einheiten cfu/ml). 5 ml dieser Zell-Suspension werden auf je eine N-Agar-Platte (mit einem Zusatz von 3% hydrolysiertem Casein und 1% Glukose) überschwemmt und 48 Stunden bei 37°C bebrütet. Danach wird das Zell-Schleim-Gemisch mit sterilen Glasspateln von der Agar-Oberfläche abgestrichen, in sterilen Glasgefäßen gesammelt und mechanisch homogenisiert (Ultraturax, Fa. Janke u. Kunkel, Staufen).

b) Vorkultur des Staphylokokken-Stammes in m S 110-Bouillon über Nacht bei 37°C. Aus dieser Vorkultur werden Gibco-Flaschen mit je 25 ml m S 110-Bouillon beimpft (0.2 ml der Vorkultur) und 18 Stunden bei 37°C bebrütet. Nach kräftigem Schütteln der Flaschen werden in eine m S 110-Agar-Platte mit 8 ml der Suspension (Kultur in Gibco-Flaschen) überschwemmt und für weitere 18 Stunden bei 37°C bebrütet. Danach wird das Zell-Schleim-Gemisch mit sterilen Glasspateln von der Agaroberfläche abgestrichen, in sterilen Glasgefäßen gesammelt und mechanisch homogenisiert.

c) Vorkultur des Staphylokokken-Stammes in BHI über Nacht bei 37°C. Die Staphylokokken-Zellen werden abzentrifugiert und dreimal in physiologischer Kochsalzlösung gewaschen und in CDM (= flüssiges, vollsynthetisches, chemisch definiertes Medium) resuspendiert ($\mapsto \sim 10^8$ cfu/ml). Daraus werden je 25 ml CDM in einer Gibco-Flasche mit 0,2 ml beimpft. Die beimpften Kulturen werden 6 Stunden bei 37°C geschüttelt. Danach wird das Zell-Schleim-Gemisch mechanisch homogenisiert.

In allen drei Fällen werden die homogenisierten Zell-Schleim-Gemische zentrifugiert (12.000 x g für 30 Minuten), um die Bakterien (Sediment) abzutrennen. Der Supernatant wird 48 Stunden extensiv gegen Aqua dest. dialysiert (mehrfaches Wasserwechseln), durch Ultrafiltration vorkonzentriert und schließlich lyophilisiert. Das vorliegende Lyophilisat ist die rohe extrazelluläre Schleimsubstanz.

Das Verfahren "a)" hat den Vorteil, daß die Ausbeute sehr hoch ist. Der Nachteil ist die Kontamination mit anderen Staphylokokken-Produkten sowie z.T. auch hochmolekularen Nährmedien-Bestandteilen z.B. Proteinen.

Beim Verfahren "b)" ist der Schleimgewinn ebenfalls sehr hoch. Ein kleiner Vorteil gegenüber "a)" ist, daß ein Dialysat-Medium verwendet wird, so daß die Kontamination mit Nährstoffen geringer ist. Das Verfahren "c)" bietet den Vorteil, daß keine Kontamination aus dem Medium möglich ist, da es voll synthetisch ist und keine Proteine und Polysaccharide enthält. Da es nur reine Substanzen wie Einzelzucker, Aminosäuren, Metalle und Vitamine enthält, können diese herausdialysiert werden. Durch die kurze Wachstumszeit ist weiterhin die Kontamination durch Produkte von schon lysierten (= abgestorbenen) Staphylokokken-Zellen sehr viel geringer. Ingesamt ist der so gewonnene Schleim von vornherein sauberer. Ein Nachteil ist, daß die Ausbeute erheblich geringer ist. Die immunmodulierende und die Proliferation von malignen Zellen hemmender Aktivität ist jedoch bei allen drei Schleimen in gleicher Weise vorhanden, so daß ein Einfluß durch das Nährmedium inzwischen ausgeschlossen werden kann.

## Auftrennung der Schleimsubstanz

a) Lyophilisierte rohe Schleimsubstanz wird in 0.15 molare NaCl (20 mg/ml) aufgelöst und mit Phenol-Kochsalz bei 65°C extrahiert (modifiziert nach Westphal). Die wässrige Phase wird gesammelt, über vier Tage gegen Leitungswasser dialysiert, über einen weiteren Tag gegen Aqua dest. dialysiert und dann gefriergetrocknet.

b) 30 mg dieses Lyophilisates wird in 2 ml Phosphatpuffer (o,1 M; pH 5,5) gelöst und auf eine DEAE-Sepharose Cl-6B-Säule (äquilibriert mit dem gleichen Phosphatpuffer) aufgetragen. Die Elution von der Säule erfolgt mit einem Kochsalzgradienten von 0,2 M bis 1,0 M und Portionen von 1 ml Volumen werden gewonnen. Durch Absorptionsmessung (Beckmann 25 Spectrophotometer, 220 nm) können 4 Peaks festgestellt werden. Die die Peaks repräsentierenden 1 ml-Portionen werden gepolt, über 48 Stunden bei 4°C gegen Auqa dest. dialysiert, durch Ultrafiltration (Amiconfilter) konzentriert und schließlich lyophilisiert. Auf diese Weise lassen sich vier unterschiedliche Fraktionen gewinnen.

## Analytik der Fraktion Nr. 4

Mit Hilfe der Gas-Flüssigkeits-Chromatographie wurden die Monosaccharid-Konstituenten der lyophilisierten Fraktion gemessen. Es wurden folgende Werte gefunden:

|  |  | µg/mg |  |
|---|---|---|---|
| Galaktose: |  | 375,67 |  |
| Ribose: |  | 56,34 | " |
| Glukose: |  | 29,42 | " |
| Aminozucker: | (Glc NAc/Gal NAc) | 17,80 |  |
| Glukuronsäure: |  | 176,63 |  |

Diese Ergebnisse unterscheiden sich qualitativ und quantitativ von den anderen Fraktionen.

Die Fraktion zeigt eine positive, spezifische Präzipitationsreaktion mit Poly-L-Lysin (Doppeldiffusionstest). Keine Präzipitation wurde beobachtet mit Mannose-spezifischen Lektinen (Concanavalin A, Lens culinaris, Pisum sativum), wie bei zwei anderen Fraktionen. Die ursprünglich eingesetzte Schleimsubstanz läßt sich prinzipiell auch mit anderen Mitteln und in andere Fraktionen aufteilen, die sich dementsprechend auch analytisch anders verhalten. Übereinstimmend wurde jedoch gefunden, daß die immunmodulierende Wirksamkeit stets in der Fraktion zu finden war, die mit Poly-L-Ly-

sin positiv reagiert.

## B e i s p i e l 2

Nachweis der Wirksamkeit der Fraktion 4.

a) Wirkung auf humane periphere mononukleäre Zellen (überwiegend T-Lymphozyten)

### 1. Verfahren

Die Isolation der mononukleären Zellen aus dem peripheren Blut erfolgt mit der Ficoll-HyPaque Dichtegradienten-Zentrifugation. $10^5$ mononukleäre Zellen wurden in Eagle's medium (Gibco, Grand Island, NY, USA; mit 100 units/ml Penicillin, 100 µg/ml Streptomycin und 2 mmol/ml L-Glutamin mit 5% fetalem Kälberserum) in Vertiefungen von Flachboden-Mikrotiterplatten inkubiert. In je drei Vertiefungen wurden entweder 100 mg Phytohämagglutinin (PHA; HA 16, Wellcome) oder 4 mg "streptococcal blastogen A" sowie verschiedene Mengen (0,1 µg - 1000 µg) der Fraktion 4 des Schleimes hinzugegeben. Diese Zellkulturen wurden für fünf Tage bei 37°C in einer 5% $CO_2$-Atmosphäre bebrütet. Danach wurde jeweils 0.5 µCi·$^3$H-Thymidin (spez. Aktivität 6.7 Ci/mmol) hinzugegeben, 18 Stunden weiterbebrütet und die Zellen geerntet. Die Menge der inkorporierten Radioaktivität wurde im Szintillationszähler gemessen.

Parallel zum $^3$H-Thymidin-Uptake wurde die Anzahl der Blasten (proliferierende Zellen) mittels Phasen-Kontrast-Mikroskopie ermittelt und mit der Anzahl in Kontroll-Kulturen ohne Fraktion 4 des Schleims verglichen.

Der Vitalitäts-Nachweis der Zellen wurde mit Hilfe der Trypan-Blau-Farbstoff-Exklusion geführt. Gleichlautende Paralelluntersuchungen wurden durchgeführt, bei denen die Fraktion 4 des Schleimes zu verschieden späten Zeitpunkten des Versuchsansatzes hinzugefügt wurde und so verschieden lang in den Zellkulturen präsent war.

## 2. Ergebnisse

Die lymphoproliferative Antwort peripherer mononukleärer Zellen nach Stimulation mit polyklonalen Mitogenen wurde in einer Dosis-Wirkungsbeziehung gehemmt. D.h. bei steigenden Dosen der Fraktion 4 des Schleimes kam es zu einer zunehmenden Proliferationshemmung. Weiterhin war die Präsenz-Zeit der Fraktion 4 im Testsystem wichtig: Je länger die Fraktion 4 anwesend war, desto ausgeprägter war die Hemmung der lymphoproliferativen Antwort. Der genaue Wirkmechanismus ist bisher ungeklärt.

b) Wirkung auf humane maligne-proliferierende Zellen aus einer Plasmozytom-Zell-Linie.

## 1. Verfahren

Die Verfahren zum Nachweis der Proliferationshemmung waren gleich denen unter a) 1 beschrieben, mit der Ausnahme, daß anstelle humaner peripherer mononukleärer Zellen Plasmozytom-Zellen verwendet wurden.

## 2. Ergebnisse

Die Proliferation von Plasmozytom-Zellen wurde in einer Dosis-Wirkungsbeziehung gehemmt.

Für die Herstellung der rohen Schleimsubstanz sind prinzipiell alle schleimbildenden Staphylokokken geeignet. Vorzugsweise werden Stämme aus der Gruppe Staphylococcus epidermidis verwendet. Die obigen Beispiele wurden mit den Stämmen ATCC 12228, sowie den Stämmen Staphylococcus epidermidis KH 11 (DSM 3269) KH 6 und V 2 (DSM 3270) durchgeführt. KH 11, KH 6 und V 2 sind die Laborbezeichnungen von selbst isolierten schleimbildenden Stämmen.

0157371

## Patentansprüche

1. Arzneimittel mit immunmodulierender und die Proliferation von malignen Zellen hemmender Wirkung enthaltend eine im Schleim von Staphylokokken vorhandene extrazelluläre Substanz.

2. Arzneimittel gemäß Anspruch 1, dadurch gekennzeichnet, daß die extrazelluläre Substanz in einer Fraktion des Schleimes vorhanden ist, die Galaktose, Ribose, Glukose, Aminozucker und Glukuronsäure enthält, eine positive, spezifische Präzipitationsreaktion mit Poly-L-Lysin, aber keine Präzipitation mit Mannose-spezifischen Lektinen aufweist.

3. Verfahren zur Herstellung einer im Schleim von Staphylokokken vorhandenen extrazellulären Substanz, dadurch gekennzeichnet, daß die lyophilisierte rohe Schleimsubstanz

a) in Kochsalzlösung gelöst und mit Phenol-Kochsalz bei 65°C extrahiert wird,

b) die wässrige Phase dialysiert und lyophilisiert in Phosphatpuffer gelöst, auf einer DEAE-Sepharose Cl-6B-Säule mit einem Kochsalzgradienten eluiert wird, und

c) die letzte (4.) bei 220 mm absorbierende Fraktion getrennt aufgefangen, dialysiert, ultrafiltriert und lyophilisiert wird.

4. Im Schleim von Staphylokokken vorhandene extrazelluläre Substanz mit immunmodulierender und die Proliferation von malignen Zellen hemmender Wirkung, erhältlich aus einer Fraktion des Schleims, die Galaktose, Ribose, Glukose, Aminozucker und Glukuronsäure enthält, eine positive, spezifische Präzipitationsreaktion mit Poly-L-Lysin, aber keine Präzipitation mit Mannose-spezifischen Lektinen aufweist.

5. Im Schleim von Staphylokokken vorhandene extrazelluläre Substanz mit immunmodulierender und die Proliferation von malignen Zellen hemmender Wirkung, dadurch erhältlich, daß die lyophilisierte rohe Schleimsubstanz

a) in Kochsalzlösung gelöst und mit Phenol-Kochsalz bei 65°C extrahiert wird,

b) die wässrige Phase dialysiert und lyophilisiert in Phosphatpuffer gelöst, auf einer DEAE-Sepharose Cl-6B-Säule mit einem Kochsalzgradienten eluiert wird, und

c) die letzte (4.) bei 220 nm absorbierende Fraktion getrennt aufgefangen, dialysiert, ultrafiltriert und lyophilisiert wird.

6. Verwendung der im Schleim von Stapholokokken vorhandenen extrazellulären Substanz gemäß Ansprüchen 4 oder 5, zur Herstellung von Arzneimitteln mit immunmodulierender und die Proliferation von malignen Zellen hemmender Wirkung.